# EUROPEAN PATENT SPECIFICATION

(11) **EP 1 762 239 B1**
(45) Date of publication and mention of the grant of the patent: **26.05.2010**
(21) Application number: 06254677.5
(22) Date of filing: 07.09.2006
(51) Int. Cl.: A61K 31/4164, A61P 25/04

(54) **Lofexidine for intraspinal administration**
Lofexidine zur intraspinalen Anwendung
Loféxidine pour l'administration par voie intraspinale

(30) Priority: 08.09.2005 EP 05255512
(43) Date of publication of application: 14.03.2007
(73) Proprietor: Texcontor Etablissement, 9490 Vaduz (LI)
(72) Inventor: Romero, Antonio, 24.08100 Mollet del Valles, Barcelona (ES)
(74) Representative: Campbell, Neil Boyd

(56) References cited:
- DOBSON M Y ET AL: "Alpha-2-Adrenoceptor component of lofexidine, clonidine and guanabenz analgesia" EUROPEAN JOURNAL OF PHARMACEUTICAL SCIENCES, vol. 4, no. SUPPL., 1996, page S54, XP009075885 & THIRD EUROPEAN CONGRESS OF PHARMACEUTICAL SCIENCES; EDINBURGH, SCOTLAND, UK; SEPTEMBER 15-17, 1996 ISSN: 0928-0987
- KINDRED A D ET AL: "Alpha-2 adrenergic agonists: A new role in outpatient anesthesia" PROGRESS IN ANESTHESIOLOGY 2004 UNITED STATES, vol. 18, no. 21, 2004, pages 347-360, XP009075857 ISSN: 0891-5784
- PUKE MALCOLM J C ET AL: "The spinal analgesic role of alpha-2-adrenoceptor subtypes in rats after peripheral nerve section" EUROPEAN JOURNAL OF PHARMACOLOGY, vol. 260, no. 2-3, 1994, pages 227-232, XP002410920 ISSN: 0014-2999
- ALGUACIL L F ET AL: "Alpha-2 adrenoceptor ligands and opioid drugs: Pharmacological interactions of therapeutic interest" CURRENT NEUROPHARMACOLOGY 2004 NETHERLANDS, vol. 2, no. 4, 2004, pages 343-352, XP001248416 ISSN: 1570-159X
- WILFFERT B ET AL: "INTERFERENCE OF ENANTIOMERS OF LOFEXIDINE WITH ALPHA-ADRENOCEPTORS" ARCHIVES INTERNATIONALES DE PHARMACODYNAMIE ET DE THERAPIE, vol. 273, no. 1, 1985, pages 18-32, XP009075886 ISSN: 0003-9780
- STONES R WILLIAM ET AL: "Randomized placebo controlled trial of lofexidine hydrochloride for chronic pelvic pain in women" HUMAN REPRODUCTION (OXFORD), vol. 16, no. 8, August 2001 (2001-08), pages 1719-1721, XP002410921 ISSN: 0268-1161
- NORIKO SHIMODE ET AL.: "The effects of..." NEUROSCIENCE LETTERS, vol. 343, 2003, pages 45-48,
- GOWING L R ET AL.: "alpha2-Adrenergic agonists..." ADDICTION, vol. 97, 2002, pages 49-58,

## Description

This invention relates to the use of lofexidine or pharmaceutically acceptable salts thereof in a method of treating a patient in need of analgesia. More particularly the invention relates to a method of providing analgesia by administering lofexidine or a pharmaceutically acceptable salt thereof intraspinally.

Lofexidine, 2-[1[(2,6-Dichlorophenoxy)ethyl]-4,5-dihydro-1H-imidazoline (CAS No. 31036-80-3) has the structure shown in formula I below:

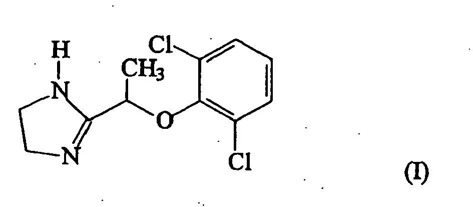

It is commercially available as its hydrochloride salt (CAS No. 21498-08-8) under the tradename Britlofex (Britannia Pharmaceuticals Ltd, UK). Alternatively

lofexidine may be synthesised according to the methods described in US 3,966,757.

The most common medicinal use of lofexidine to date has been to alleviate the symptoms of drug withdrawal (e.g. withdrawal from alcohol, tobacco, heroin, opium use etc) and, in the UK, lofexidine hydrochloride has been granted Regulatory Approval for this application. Other uses of lofexidine have also been disclosed. US 4,312,879 discloses use of lofexidine for treatment of diarrhoea, US 5,863,934 describes use of lofexidine to improve behavioural inhibition in patients with attention deficiency disorder and conduct disorder and US 4,532,135 discloses use of lofexidine in protective or prophylactic treatment against renal failure.

There has been some discussion of the alpha-2 adrenoceptor component of lofexidine in, for example, Dobson M. Y. et al, "Alpha-2-Adrenoceptor component of lofexidine, clonidine and guanabenz analgesia", Eur. J. Pharma. Sci., Vol. 4 (1996), Page S54; Kindred D. A. et al, "Alpha-2 adrenergic agonists: A new role in outpatient anesthesia", Prog. Anesth. 2004 US, Vol. 18, No. 21 (2004), pages 347-360; Puke M. J. et al, "The spinal analgesic role of alpha-2-adrenoceptor subtypes in rats after peripheral nerve section", Eur. J. Pharma., Vol. 260, No. 2-3 (1994), pages 227-232; Alguacil L. F. et al, "Alpha-2 adrenoceptor ligands and opioid drugs: Pharmacological interactions of therapeutic interest", Curr. Neuropharma. 2004 NL, Vol. 2, No. 4 (2004), pages 343-352; and Wilffert B. et al, "Interference of enantiomers of lofexidine with alpha adrenoceptors", Archives Internationale de Pharmacodynamie et de Therapie, Vol. 273, No. 1 (1985), pages 18-32.

A few publications have also disclosed use of lofexidine to provide pain relief. For example, US 6,417,184 describes a pharmaceutical composition containing, amongst other active ingredients, lofexidine for treatment of acute and chronic pain symptoms. Additionally WO99/55334 to Britannia Pharmaceuticals Ltd. suggests use of 200-600 µg of lofexidine (preferably orally) for treatment of chronic pelvic pain. However, in a randomised placebo-controlled parallel group trial undertaken to investigate the usefulness of lofexidine in this application the conclusion reached was that lofexidine hydrochloride given orally is not effective for this treatment (Human Reproduction, Vol. 16, No. 8, pp. 1719-1721, 2001). This was despite the use of dosages of up to 1200 µg per day.

The usefulness of lofexidine in providing pain relief is therefore uncertain. Moreover it has been reported that the administration of lofexidine, and especially lofexidine hydrochloride, systemically (e.g. orally or by intravenous injection) has some major drawbacks, especially at high doses (e.g. >600 µg per day). Common symptoms associated with use of lofexidine hydrochloride in this way include drowsiness, sedation and blood pressure and heart rate increases. As a result, lofexidine hydrochloride is unattractive for use in certain medical applications and is contraindicated in, for example, patients with high risk of cardiovascular disease.

To date all uses of lofexidine as a pharmaceutical have involved systemic administration and nowhere is it suggested that administration of lofexidine intraspinally may be advantageous. However it has now been surprisingly found that intraspinal administration of lofexidine or pharmaceutically acceptable salts thereof (e.g. lofexidine hydrochloride) can be used in a method of providing analgesia. In particular it has surprisingly been found that administration of lofexidine or pharmaceutically acceptable salts thereof intraspinally can provide analgesia without adverse side affects such as sedation.

Thus, viewed from one aspect the invention provides lofexidine or a pharmaceutically acceptable salt thereof for use in intraspinal administration to provide analgesia, wherein said analgesia is for post-surgical pain and/or neuropathic pain.

In a further aspect the invention provides the use of lofexidine or a pharmaceutically acceptable salt thereof. In the manufacture of a medicament for intraspinal administration to provide analgesia, wherein said analgesia is for post-surgical pain and/or neuropathic pain.

As used herein, the term lofexidine is intended to include lofexidine, pharmaceutically acceptable salts of lofexidine and mixtures thereof.

As used herein, the term intraspinally is intended to include epidural, intrathecal (i.e. within the spinal subarachnoid or subdural space) and inrarrhachidian administration. Epidural and intrathecal administration are particularly preferred, especially intrathecal administration. Administration may be carried out using a syringe or catheter. Use of a syringe is preferred.

As used herein, the term analgesia denotes provision of pain relief without causing loss of consciousness. The compounds described herein may be used to provide analgesia in the treatment of post-operative pain, and neuropathic pain (e.g. as is often encountered during cancer). Intraspinally administered lofexidine or pharmaceutically acceptable salts thereof are particularly effective in providing analgesia for post-operative pain and neuropathic pain.

At present, opioids such as morphine, are routinely used for intraspinal administration to give analgesia. However, a major concern associated with the use of such compounds is the 0.1 to 0.2 % incidence of severe respiratory depression occurring six to twelve hours after injection. Other undesirable side effects include pruritus, tolerance, dependence and constipation. The use of lofexidine or pharmaceutically acceptable salts thereof as described herein therefore provides an attractive alternative or adjunct to this conventional treatment. This is especially the case since spinally administered lofexidine or pharmaceutically acceptable salts thereof will provide analgesia without severe side affects, e.g. with only limited sedation, blood pressure effects and/or drowsiness.

The lofexidine and lofexidine salts for use according to the invention may be in the form of a free amine (i.e. -NH-) or more preferably in the form of a pharmaceutically acceptable salt. Such salts are preferably acid addition salts with physiologically acceptable organic or inorganic acids. Suitable acids include, for example, hydrochloric, hydrobromic, phosphoric, sulphuric and sulphonic acids. Particularly preferred salts are acid addition salts with hydrochloric acid. Procedures for salt formation are conventional in the art.

Lofexidine and its salts occur in two different enantiomeric forms. Although lofexidine may be used in racemic form, the lofexidine for use in the invention is preferably enantiomerically pure (e.g. it has an enantiomeric excess of at least 90 %, more preferably at least 95 %, still more preferably at least 99 % by weight). The preferred enantiomer for use in the invention is (-)-lofexidine. Similarly, preferred pharmaceutically acceptable salts of lofexidine are those formed from (-)-lofexidine. Enantiomerically pure lofexidine and pharmaceutically acceptable salts thereof may be prepared by conventional procedures described in the art (e.g. as described in J. Med. Chem., 1986, 29, 991183-1188).

Lofexidine and pharmaceutically acceptable salts thereof for use according to the invention may be formulated in any conventional manner with one or more physiologically acceptable carriers and/or diluents (e.g. sterile saline solution) according to techniques known in the art. Suitable concentrations of lofexidine or lofexidine salt in such formulations may be readily determined by those skilled in the art. Typical formulations may have a lofexidine/lofexidine salt concentration of 0.0032-0.32 mg/ml, more preferably 0.01-0.032 mg/ml, e.g. about 0.2 mg/ml. These concentrations may, however, be modified by those skilled in the art as necessary, e.g. decreased in formulations for use in patient-controlled analgesia.

The formulations for use in the invention may be prepared immediately before use. More preferably the formulations may be pre-prepared and stored in a form ready for intraspinal administration. The formulations may, for example, be stored in containers which can be penetrated by a syringe.

Viewed from a yet further aspect the invention provides an intraspinal syringe (e.g. an intrathecal or epidural syringe) containing lofexidine or a pharmaceutically acceptable salt thereof with one or more physiologically acceptable carriers and/or diluents for use in intraspinal administration to provide analgesia wherein said analgesia is for post-surgical pain and or nanopathic pain.

Particularly preferred formulations for use in the invention comprise lofexidine or pharmaceutically acceptable salts thereof in liposome-encapsulated form. Liposome encapsulation of lofexidine and salts thereof may be carried out by any conventional procedure known in the art, e.g. by sonication. Specific examples of encapsulation procedures are described in Liposome Technology (CRC Press, Boca Raton. FL, USA, 1st Edition), 1984, Vol. 1, pp 19-27 and Liposome Technology (CRC Press, Boca Raton. FL, USA, 2nd Edition), 1993, Vol. I, pp 99-110. Liposome-encapsulated lofexidine/lofexidine salts may advantageously provide prolonged analgesia without the need for repeated injections and/or use of an intraspinal catheter. In preferred formulations for use in the present invention lofexidine or salts thereof are encapsulated in pharmaceutically acceptable phosphatidyl choline-based liposomes.

Further preferred formulations for use in the invention comprise an additional active substance, e.g. an opioid analgesic. Representative examples of opioid analgesics include morphine, fentanyl, alfentanil, sulfentanil, meperidine, levorphanol, hydromorphone, oxycodone, oxymorphone and pentazocine. Preferred opioid analgesics include morphine and fentanyl. An advantage associated with the use of lofexidine or a pharmaceutically acceptable salt thereof in conjunction with a further active substance (e.g. an opioid analgesic) is the dose of one or both of the compounds may be lowered. This is particularly advantageous when the further active substance is associated with known side effects (e.g. as is the case with opioid analgesics).

The quantity of lofexidine or a pharmaceutically acceptable salt thereof and optional further active substance(s) present in the formulations for use in the invention may be readily determined by those skilled in the art and will depend on several factors, including the weight of the subject, the level of analgesia required and the nature of any further optional active substance(s). Generally, however, lofexidine or pharmaceutically acceptable salts thereof may be used to provide pain relief in adult humans in amounts of 30 to 500 µg/patient/dose, more preferably 40 to 200 µg/patient/dose, e.g. about 50 to 100 µg/patient/dose. Typical amounts of further active substances present in the formulations for use in the invention are: 0.1-2mg/patient/dose morphine and 0.0032-0.1 mg/patient/dose fentanyl. One or more (e.g. two or three) doses may be administered per day.

The invention will now be described in more detail in the following nonlimiting examples.

### Example 1

Lofexidine at 0.00032 and 0.0032 mg/rat injected intrathecally (ITH) was evaluated for possible analgesic activity in a rat model of post-operative pain which was induced with an incision made on the plantar surface of the hind paw. To measure tactile allodynia, a supertip with a flattened surface connected to Electrovonfrey Aesthethiometer (IITC, USA) was applied slowly and gently onto the plantar surface of the hind paw, where 4 points around the incision were identified. The endpoint is the mean withdrawal threshold values (gm) in response to von Frey filament, serving as a measure of analgesic activity. Percent inhibition was calculated as Δ treatment values (change in threshold pressure caused by treatment) relative to Δ blank values (change in threshold value caused by incision).

### Experimental

### 1. Test Substances and Dosing Pattern

Lofexidine.HCl was dissolved in 0.9% NaCl and at doses of 0.00032 and 0.0032 mg/rat was administered intrathecally (ITH). The dosing volume was 10 µl/rat.

### 2. Animals

Male Wistar derived rats weighing 270 ± 30 g provided by BioLasco Taiwan (under Charles River Laboratories Technology licensee) were used. Space allocation for animals was 45 x 23 x 15 cm for 6 rats. Animals were housed in APEC^{R} cages. All animals were maintained in a controlled temperature (22-24°C) and humidity (60%-80%) environment with 12 hours light dark cycles for at least one week in MDS Pharma Services-Taiwan Laboratory prior to use. Free access to standard lab chow (Lab Diet, Rodent Diet, PMI Nutrition International, USA) and tap water was granted. All aspects of this work including housing, experimentation and disposal of animals were performed in general accordance with the Guide for the Care and Use of Laboratory Animals (National Academy Press, Washington, D. C, 1996).

### 3. Chemicals

0.9% NaCl (Sintong, R. O. C).

### 4. Equipment

4-0 Silk suture, Needle holder (Klappencker, Germany), Animal case (ShinTeh, R. O. C), Electronic Von Frey Anesthesiometer (2290CE Electrovonfrey, IITC, USA), Stainless Forceps (Klappencker, Germany), Hypodermic needle (23 G x 1", Top, Japan), Mosquito hemostat (Klappencker, Germany), Rat Scale 500 g (Chien-Chun, R. O. C), Rigid tip (IITC, USA), Stainless Scissors (Klappencker, Germany), Surgical blade (Feather, Japan), Syringe Glass 2 ml (Mitsuba, Japan), Stop-Watch (Heuer, Switzerland) and Wire mesh Rack (in-house, R. O. C).

### 5. Procedure

Groups of 5 male Wistar rats weighing 270 ± 30 g were used. Prior to surgery, individual animals were acclimatised to the testing environment and baseline responses to Electronic Von Frey Aesthesiometer (2290CE Electrovonfrey®, IITC, USA) with a rigid tip were performed (Pre-Incision). Under pentobarbital (50 mg/kg, 5 ml/kg, i.p.) anesthesia, the left hind paw was aseptically prepared. A 1-cm longitudinal incision was made with a number 11 blade, through the skin and fascia of the plantar aspect of the foot, starting 0.5 cm from the proximal edge of the heel and extending toward the toes. The *plantaris* muscle was elevated and incised longitudinally, followed by crushing with a mosquito hemostat for 20 seconds. The muscle origin and insertion remained intact After hemostasis with gentle pressure, the skin and fascia were then closed as one layer using 4-0 silk suture.

Evaluation for pain was performed at 24 hours postoperatively between 08:00 and 16:00h. Each animal was monitored at 15, 30, 60 and 120 minutes after intrathecal administration of lofexidine.HCl. The rats were placed under inverted plexiglas cages on a wire mesh rack and a brief observation of general behavior post-injection was made. The rigid tip part of the Electronic Von Frey Aesthesiometer was applied perpendicularly from underneath the mesh floor to the four points around the wound surface of the animal with slow upward force against the paw. A positive response to tactile pressure was recorded if the paw was sharply withdrawn; ambulation was considered an ambiguous response, and in such cases, the stimulus was reapplied. Mean values from 4 points for each rat and from 5 rats in each group were calculated. Percent inhibition due to treatment relative to pre-treatment pain response was calculated according to the formula: Tactile Pressure [(Post-Treatment) - (Pre-Treatment)]/[(Pre-Incision) - (Pre-Treatment)] x 100%. Inhibition by 50% or more (≥50%) is considered significant analgesic effect. Unpaired Student's *t* test is applied for comparison of the % inhibition values between test substance-and vehicle-treated groups. Significance is considered at P<0.05 level

### Results:

The results are shown in Tables 1-3 below.

**Table 1**

| | | (24 Hours After Surgery) | | | | | | | |
|---|---|---|---|---|---|---|---|---|---|
| Treatment | Route | Dose | N | Tactile Pressure (g) | | | | | |
| | | | (B.W.) | Pre- | Pre- | Post-treatment | | | |
| | | | | Incision | Treatment | 15min. | 30min. | 60min. | 120 min. |
| Veh.0.9%NaCl | ITH | 0.00032 mg/rat | 1 | 57.1 | 22.7 | 23.1 | 19.0 | 22.5 | 21.1 |
| | | | (295 g) | 43.9 | 21.8 | 22.3 | 21.6 | 19.6 | 22.3 |
| | | | | 41.8 | 19.7 | 22.2 | 20.6 | 22.5 | 19.9 |
| | | | | 60.1 | 25.4 | 22.9 | 20.1 | 21.5 | 22.8 |
| | | | Average | 50.7 | 22.4 | 22.6 | 20.3 | 21.5 | 21.5 |
| | | | %Inh. | | | 0.7 | -7.4 | -3.2 | -3.2 |
| | | | 2 | 46.1 | 23.6 | 24.8 | 21.9 | 22.4 | 21.8 |
| | | | (280 g) | 56.1 | 23.5 | 20.4 | 24.9 | 19.2 | 20.0 |
| | | | | 53.6 | 20.6 | 23.6 | 25.2 | 20.6 | 22.0 |
| | | | | 59.4 | 22.8 | 24.1 | 19.8 | 24.2 | 20.7 |
| | | | Ave. | | | 23.2 | 23.0 | 21.6 | 21.1 |
| | | | %Inh | 53.8 | 22.6 | 1.9 | 1.3 | -3.2 | -4.8 |
| | | | 3 | 59.0 | 20.9 | 18.1 | 19.3 | 22.9 | 16.4 |
| | | | (256 g) | 50.5 | 15.1 | 18.2 | 15.7 | 20.2 | 18.2 |
| | | | | 53.3 | 20.9 | 23.1 | 19.2 | 20.8 | 22.0 |
| | | | | 46.9 | 19.2 | 20.0 | 16.4 | 20.8 | 15.6 |
| | | | Ave. | | | 19.9 | 17.7 | 21.2 | 18.1 |
| | | | %Inh | 52.4 | 19.0 | 2.7 | -3.9 | 6.6 | -2.7 |
| | | | 4 | 51.1 | 23.5 | 20.2 | 19.5 | 23.1 | 19.5 |
| | | | (240 g) | 46.9 | 14.5 | 18.1 | 19.7 | 18.3 | 20.2 |
| | | | | 51.1 | 20.5 | 19.3 | 17.3 | 19.1 | 23.5 |
| | | | | 52.5 | 22.7 | 17.8 | 17.9 | 21.0 | 15.7 |
| | | | Ave. | | | 18.9 | 18.6 | 20.4 | 19.7 |
| | | | %Inh | 50.4 | 20.3 | -4.7 | -5.6 | 0.3 | -2.0 |
| | | | 5 | 52.2 | 19.2 | 14.5 | 18.9 | 16.2 | 18.1 |
| | | | (265 g) | 46.3 | 24.8 | 18.4 | 24.2 | 23.1 | 18.9 |
| | | | | 53.7 | 23.1 | 22.6 | 24.4 | 23.7 | 22.5 |
| | | | | 40.5 | 15.0 | 21.5 | 20.7 | 18.2 | 22.8 |
| | | | Ave. | | | 19.3 | 22.1 | 20.3 | 20.6 |
| | | | %Inh | 48.2 | 20.5 | -4.3 | 5.8 | -0.7 | 0.4 |
| | | Mean | | 51.1 | 21.0 | 20.8 | 20.3 | 21.0 | 20.2 |
| | | SEM | | 0.9 | 0.7 | 0.9 | 1.0 | 0.3 | 0.6 |
| | | Δ Blank | | | 30.1 | | | | |
| | | Δ Treatment | | | | -0.2 | -0.7 | 0 | -0.8 |
| | | %Inhibition | | | | -0.7 | -2.3 | 0 | -2.7 |

| | | | | | | | | | |
|---|---|---|---|---|---|---|---|---|---|
| Δ Blank: Tactile Pressure [(Pre-incision) - (Pre-Treatment)] Δ Treatment: Tactile Pressure [(Post-Treatment)- (Pre-Treatment)] % Inhibition: Δ Treatment/ Δ Blank x 100% | | | | | | | | | |

**Table 2**

| | | (24 Hours After Surgery) | | | | | | | |
|---|---|---|---|---|---|---|---|---|---|
| Treatment | Route | Dose | N | Tactile Pressure (g) | | | | | |
| | | | (B.W.) | Pre- | Pre- | Post-treatment | | | |
| | | | | Incision | Treatment | 15min. | 30min. | 60min. | 120 min. |
| Lofexidine.HCl | ITH | 0.00032 mg/rat | 1 | 44.8 | 17.7 | 17.9 | 20.0 | 16.2 | 18.6 |
| | | | (252 g) | 53.2 | 21.8 | 19.2 | 19.4 | 21.1 | 16.8 |
| | | | | 40.9 | 24.0 | 14.4 | 182 | 20.5 | 22.4 |
| | | | | 55.3 | 16.9 | 20.6 | 192 | 21.6 | 19.9 |
| | | | Average | 48.6 | 20.1 | 18.0 | 192 | 19.9 | 19.4 |
| | | | %Inh. | | | -7.4 | -32 | -0.7 | -2.5 |
| | | | 2 | 48.0 | 21.3 | 26.6 | 25.9 | 23.3 | 20.6 |
| | | | (285 g) | 54.8 | 27.8 | 26.9 | 23.6 | 27.3 | 21.0 |
| | | | | 51.3 | 19.6 | 28.6 | 32.5 | 23.6 | 17.2 |
| | | | | 51.8 | 19.0 | 23.1 | 18.6 | 26.6 | 19.9 |
| | | | Ave. | 51.5 | 21.9 | 26.3 | 25.2 | 252 | 19.7 |
| | | | %Inh | | | 14.9 | 11.1 | 11.1 | -7.4 |
| | | | 3 | 53.4 | 21.3 | 21.6 | 25.7 | 22.9 | 24.3 |
| | | | (285 g) | 59.2 | 20.0 | 17.6 | 28.4 | 19.7 | 21.2 |
| | | | | 57.3 | 19.1 | 23.3 | 26.0 | 24.4 | 21.0 |
| | | | | 58.6 | 21.4 | 23.1 | 20.6 | 19.2 | 18.9 |
| | | | Ave. | 57.1 | 20.5 | 21.4 | 25.2 | 21.6 | 21.4 |
| | | | %Inh | | | 2.5 | 12.8 | 3.0 | 2.5 |
| | | | 4 | 40.5 | 20.4 | 15.5 | 22.3 | 19.9 | 21.6 |
| | | | (275 g) | 51.2 | 22.7 | 18.4 | 20.4 | 20.3 | 22.0 |
| | | | | 41.1 | 16.4 | 21.1 | 17.3 | 22.6 | 20.6 |
| | | | | 54.1 | 21.0 | 19.3 | 24.6 | 18.4 | 20.6 |
| | | | Ave. | 46.7 | 20.1 | 18.6 | 21.2 | 20.3 | 21.2 |
| | | | %Inh | | | -5.6 | 4.1 | 0.8 | 4.1 |
| | | | 5 | 47.9 | 19.9 | 23.6 | 21.6 | 25.1 | 18.0 |
| | | | (290 g) | 43.8 | 18.7 | 20.5 | 22.5 | 21.2 | 20.3 |
| | | | | 49.7 | 18.4 | 24.6 | 24.4 | 22.0 | 22.4 |
| | | | | 57.3 | 22.3 | 20.1 | 24.3 | 19.7 | 23.9 |
| | | | Ave. | 49.7 | 19.8 | 22.2 | 23.2 | 22.0 | 21.2 |
| | | | %Inh | | | 8.0 | 11.4 | 7.4 | 4.7 |
| | | P value | | | | 0.4904 | 0.0447* | 0.1611 | 02962 |
| | | Mean | | 50.7 | 20.5 | 21.3 | 22.8* | 21.8 | 20.6 |
| | | SEM | | 1.8 | 0.4 | 1.5 | 1.2 | 0.9 | 0.4 |
| | | Δ Blank | | | 30.2 | | | | |
| | | Δ Treatment | | | | 0.8 | 2.3 | 1.3 | 0.1 |
| | | %Inh | | | | 2.6 | 7.6 | 4.3 | 0.3 |

| | | | | | | | | | |
|---|---|---|---|---|---|---|---|---|---|
| Δ Blank Tactile Pressure [(Pre-incision) - (Pre-Treatment)] Δ Treatment: Tactile Pressure [(Post-Treatment)- (Pre-Treatment)] % Inhibition: Δ Treatment/ Δ Blank x 100% * P<0.05 vs vehicle control with individual % inhibition by unpaired Studat's test | | | | | | | | | |

**Table 3**

| (24 Hours After Surgery) | | | | | | | | | |
|---|---|---|---|---|---|---|---|---|---|
| Treatment | Route | Dose | N | Tactile Pressure (g) | | | | | |
| | | | (B.W.) | Pre- | Pre- | | Post-treatment | | |
| | | | | Incision | Treatment | 15 min. | 30min. | 60min. | 120 min. |
| Lofexidine.HCl | ITH | 0.0032 mg/rat | 1 | 44.0 | 24.7 | 40.7 | 27.7 | 31.3 | 31.1 |
| | | | (270 g) | 56.8 | 20.9 | 60.0 | 44.2 | 41.3 | 28.6 |
| | | | | 51.8 | 16.4 | 42.5 | 54.3 | 32.5 | 27.9 |
| | | | | 50.9 | 17.1 | 40.6 | 50.8 | 49.5 | 28.7 |
| | | | Ave. | 50.9 | 19.8 | 46.0 | 44.3 | 38.7 | 29.1 |
| | | | %Inh | | | 84.2 | 78.8 | 60.8 | 29.9 |
| | | | 2 | 53.2 | 24.7 | 48.4 | 36.4 | 33.6 | 29.3 |
| | | | (265 g) | 51.2 | 222 | 47.2 | 34.8 | 35.6 | 21.6 |
| | | | | 52.5 | 19.8 | 55.3 | 44.1 | 35.5 | 20.4 |
| | | | | 68.4 | 22.2 | 30.4 | 39.3 | 36.7 | 19.2 |
| | | | Ave. | 56.3 | 22.2 | 45.3 | 38.7 | 35.4 | 22.6 |
| | | | %Inh | | | 67.7 | 48.4 | 38.7 | 1.2 |
| | | | 3 | 55.9 | 20.6 | 23.5 | 34.1 | 29.0 | 22.9 |
| | | | (262 g) | 61.6 | 19.0 | 48.7 | 44.0 | 28.5 | 22.1 |
| | | | | 55.5 | 18.3 | 43.0 | 38.1 | 40.6 | 21.4 |
| | | | | 63.3 | 20.9 | 36.2 | 36.5 | 31.8 | 21.6 |
| | | | Ave. | 59.1 | 19.7 | 37.9 | 38.2 | 32.5 | 22.0 |
| | | | %Inh | | | 46.2 | 47.0 | 32.5 | 5.8 |
| | | | 4 | 42.9 | 18.2 | 32.4 | 36.8 | 16.1 | 14.5 |
| | | | (240 g) | 43.8 | 20.4 | 33.8 | 28.8 | 16.5 | 21.5 |
| | | | | 53.7 | 17.8 | 21.4 | 30.3 | 21.8 | 17.6 |
| | | | | 47.9 | 18.6 | 31.9 | 332 | 16.6 | 18.8 |
| | | | Ave. | 47.1 | 18.8 | 29.9 | 32.3 | 17.8 | 18.1 |
| | | | %Inh | | | 39.2 | 47.7 | -3.5 | -2.5 |
| | | | 5 | 54.1 | 19.5 | 37.7 | 34.5 | 24.3 | 16.2 |
| | | | (260 g) | 56.1 | 22.2 | 42.5 | 40.1 | 26.8 | 22.6 |
| | | | | 51.3 | 252 | 34.5 | 37.2 | 21.3 | 24.7 |
| | | | | 57.1 | 25.3 | 30.5 | 26.8 | 21.1 | 18.6 |
| | | | Ave. | 54.7 | 23.1 | 36.3 | 34.7 | 23.4 | 20.5 |
| | | | %Inh | | | 41.8 | 36.7 | 0.9 | -8.2 |
| | | P value | | | | 0.0002* | <.00001* | 0.0664 | 0.2791 |
| | | Mean | | 53.6 | 20.7 | 39.1* | 37.6* | 29.6 | 22.5 |
| | | SEM | | 2.1 | 0.8 | 3.0 | 2.0 | 3.9 | 1.8 |
| | | Δ Blank | | | 32.9 | | | | |
| | | ΔTreatment | | | | 18.4 | 16.9 | 8.9 | 1.8 |
| | | %Inh. | | | | (55.9) | (51.4) | 27.1 | 5.5 |

| | | | | | | | | | |
|---|---|---|---|---|---|---|---|---|---|
| Blank Tactile Pressure [(Pre-incision) - (Pre-Treatment)] Δ Treatment: Tactile Pressure [(Post-Treatment)- (Pre-Treatment)] % Inhibition: ΔTreatment/ Δ Blank x 100%; Inhibition by 50% or more (≥50%) is considered significant analgesic effect * P<0.05 vs vehicle control with individual % inhibition by unpaired Student's *t* test | | | | | | | | | |

### Conclusion

Intrathecal administration of lofexidine at 0.0032 mg/rat demonstrated analgesic effect against post-operative pain at 15 and 30 minute time points when compared with Δ blank values. In addition, lofexidine at 0.0032 mg/rat ITH was associated with moderate 27.1% inhibition of pain response relative to Δ blank value at 60 min point. Lofexidine at 0.00032 mg/rat ITH caused a slight analgesic effect.

Furthermore, lofexidine.HCl at 0.00032 mg/rat and 0.0032 mg/rat (ITH) did not cause behavioral changes in treated animals; all treated animals assumed normal gait after intrathecal injection.

### Example 2

Lofexidine was evaluated for possible analgesic activity in neuropathic pain using a nerve ligation-induced chronic pain model in rats. Lofexidine HCl was administered intrathecally (ITH) at 3.2 and 10 µg/animal and analgesia was measured at 15, 30, 60 and 120 minutes post-dosing using an Electronic Von Frey Aesthesiometer (IITC, USA) with a #12 supertip. A 50 % or greater (≥50%) inhibition relative to blank indicates possible analgesic activity.

### 1. Test Substances and Dosing Pattern

Lofexidine HCl at doses of 3.2 and 10 µg/animal were administered intrathecally. Sterile Saline (obtained from Sintong Biotech Company, Taiwan) was used as vehicle and dosing volume was 10 µl/animal.

### 2. Animals

Male Wistar rats provided by Biolasco Taiwan (under a Charles River Laboratories Technology Licensee) were used. Space allocation for 6 animals was 45 x 23 x 21 cm. Animals were housed in APEC^{R} cages and maintained in a controlled temperature (22-23°C) and humidity (60-70%) environment with 12 hours light dark cycles for at least one week in MDS Pharma Services - Taiwan Laboratory prior to use. Free access to standard lab chow for rats (Lab Diet, Rodent Diet, PMI Nutrition International, USA) and tap water was granted. All aspects of this work including housing, experimentation and disposal of animals were performed in general accordance with the Guide for the Care and Use of Laboratory Animals (National Academy Press, Washington, D. C, 1996).

### 3. Chemicals

Isotonic Sodium Chloride Solution (0.9% NaCl, Sintong, Taiwan).

### 4. Equipment

4-0 Silk suture (UNIK, R. O. C), Wire mesh Rack (in-house, R. O. C), Supertip (IITC, USA), Electronic Von Frey Anesthesiometer (2290CE Electrovonfrey, IITC, USA), Animal Case (Allentown Caging, USA), Stainless Forcepts (Klappencker, Germany), Hypodermic needle (23 G x 1", 25 G x 1", Top, Japan), Rat Scale 500 g (Chien-Chun, R. O. C), Stainless Scissors (Klappencker, Germany), Syringe Glass 2 ml (Mitsuba, Japan) and Stop-Watch (Heuer, Switzerland).

### 5. Procedure

Male Wistar rats weighing 160 to 200 g were used. Under pentobarbital (50 mg/kg, 5 ml/kg, i.p.) anesthesia, the sciatic nerve was exposed at mid-thigh level. Three ligatures (4-0 silk suture), about 1 mm apart, were loosely tied around th e nerve. The animals were then housed individually in cages with soft bedding for 7 days before testing for mechanical allodynia. On the test day, rats were placed under inverted plexiglas cages on a wire mesh rack and allowed to acclimatise for 20 to 30 minutes. Allodynia was evaluated by responsiveness to a #12 Supertip® (IITC, USA) applied beneath the mesh floor perpendicular to the central plantar surface of the left hind paw. The tip was gradually applied with sufficient force to cause slight buckling of the filament against the paw. A positive response to the applied tactile pressure, noted by sharp withdrawal of the paw, was recorded automatically by an Electronic Von Frey Aesthesiometer (2290CE Electrovonfrey®, IITC, USA). The endpoint is the withdrawal threshold (gm) to von Frey filament, serving as a measure of analgesic activity. Rats were pre-selected (for presence of allodynia) for experimentation only if the withdrawal threshold 7 days after nerve ligation (pre-treatment) was reduced by 10 grams of force relative to the response of the individual paw before nerve ligation (pre-ligation). Test substance or vehicle was administered intrathecally (3.2 µg and 10 µg/animal) to groups of 5 animals and the level of allodynia was again determined (at 15, 30, 60 and 120 minutes post-treatment). Allodynia is calculated as follows: % Inhibition=Δ Treatment/Δ Blank x 100%; where Δ Treatment is Tactile Pressure [(Post-Treatment) -(Pre-Treatment)] and Δ Blank is Tactile Pressure [(Pre-Ligation) - (Pre-Treatment)]. A 50 percent or greater (≥50%) inhibition relative to blank indicates possible analgesic activity.

### Results:

The results are shown in Tables 4-7 below.

**Table 4: Analgesia against Neuropathic Pain (Nerve Ligation in rats)**

| | | | | | | | | | |
|---|---|---|---|---|---|---|---|---|---|
| (15 Minutes) | | | | | | | | | |

| Treatment | Route | Dose | N | Tactile Pressure (g) | | | | | Inhibition % |
|---|---|---|---|---|---|---|---|---|---|
| | | | | Pre-Ligation | Pre-Treatment | Δ Blank | Post-Treatment | Δ Treatment | |
| Vesicle | ITH | 10 µl/animal 1 | | 32.1 | 11.7 | 20.4 | 7.5 | -4.2 | |
| (Sterile | | 2 | | 34.2 | 7.6 | 26.6 | 7.4 | -0.2 | |
| Saline) | | 3 | | 37.0 | 13.3 | 23.7 | 10.6 | -2.7 | |
| | | 4 | | 40.0 | 14.2 | 25.8 | 7.9 | -6.3 | |
| | | 5 | | 46.2 | 9.2 | 37.0 | 13.0 | 3.8 | |
| | | | Average | 37.9 | 11.2 | 26.7 | 9.3 | -1.9 | -7 |
| Lofexidine HCl | ITH | 3.2 µg/animal 1 | | 32.9 | 10.7 | 22.2 | 8.9 | -1.8 | |
| | | 2 | | 34.4 | 11.2 | 23.2 | 5.8 | -5.2 | |
| | | 3 | | 40.1 | 14.8 | 25.3 | 23.3 | 8.5 | |
| | | 4 | | 48.7 | 7.1 | 41.6 | 13.4 | 6.3 | |
| | | 5 | | 50.6 | 12.0 | 38.6 | 10.9 | -1.1 | |
| | | | Average | 41.3 | 11.2 | 30.2 | 12.5 | 1.3 | 4 |
| | ITH | 10 µg/animal 1 | | 37.8 | 12.1 | 25.7 | >36.8 | 24.7 | |
| | | 2 | | 40.9 | 10.9 | 30.0 | 12.4 | 1.5 | |
| | | 3 | | 35.6 | 6.2 | 29.4 | 17.2 | 11.0 | |
| | | 4 | | 37.0 | 11.0 | 26.0 | 26.7 | 15.7 | |
| | | 5 | | 39.6 | 11.3 | 28.3 | 23.6 | 12.3 | |
| | | | Average. | 38.2 | 10.3 | 27.9 | 23.3 | 13.0 | 47 |

| | | | | | | | | | |
|---|---|---|---|---|---|---|---|---|---|
| Test substance was administered intrathecally to groups of 5 animals 15 minutes before the level of allodynia was again determined (post-treatment). 50 percent or greater (≥50%) inhibition relative to blank indicated possible analgesic activity. Note: (1) Δ Blank: Tactile Pressure [(PreLigation) - (Pre-Treatment)] (2) Δ Treatment Tactile Pressure [(Post-Treatment) - (Pre-Treatment)] (3) % Inhibition = Δ Treatment/ Δ Blank x 100% (4) ITH:Intrathecal | | | | | | | | | |

**Table 5: Analgesia against Neuropathic Pain (Nerve Ligation in rats)**

| (30 Minutes) | | | | | | | | | |
|---|---|---|---|---|---|---|---|---|---|
| Treatment | Route | Dose | N | Pre-Ligation | Tactile Pressure (g) | | | | Inhibition % |
| | | | | | Pre-Treatment | Δ Blank | Post-Treatment | Δ Treatment | |
| Vehicle | ITH | 10 µl/animal | 1 | 32.1 | 11.7 | 20.4 | 9.1 | -2.6 | |
| (Sterile | | | 2 | 34.2 | 7.6 | 26.6 | 13.0 | 5.4 | |
| Saline) | | | 3 | 37.0 | 13.3 | 23.7 | 12.3 | -1.0 | |
| | | | 4 | 40.0 | 14.2 | 25.8 | 12.1 | -2.1 | |
| | | | 5 | 46.2 | 9.2 | 37.0 | 8.5 | -0.7 | |
| | | | Av | 37.9 | 11.2 | 26.7 | 11.0 | -0.2 | -1 |
| Lofexidine HCl | ITH | 3.2µg/animal | 1 | 32.9 | 10.7 | 22.2 | 10.2 | -0.5 | |
| | | | 2 | 34.4 | 11.2 | 23.2 | 7.4 | -3.8 | |
| | | | 3 | 40.1 | 14.8 | 25.3 | 14.2 | -0.6 | |
| | | | 4 | 48.7 | 7.1 | 41.6 | 8.5 | 1.4 | |
| | | | 5 | 50.6 | 12.0 | 38.6 | 10.0 | -2.0 | |
| | | | Av | 41.3 | 11.2 | 30.2 | 10.1 | -1.1 | -4 |
| | ITH | 10 µg/animal | 1 | 37.8 | 12.1 | 25.7 | 42.6 | 30.5 | |
| | | | 2 | 40.9 | 10.9 | 30.0 | 7.0 | -3.9 | |
| | | | 3 | 35.6 | 6.2 | 29.4 | >31.5 | 25.3 | |
| | | | 4 | 37.0 | 11.0 | 26.0 | >35.5 | 24.5 | |
| | | | 5 | 39.6 | 11.3 | 28.3 | 15.6 | 4.3 | |
| | | | Av | 38.2 | 10.3 | 27.9 | 26.4 | 16.1 | 58 |

| | | | | | | | | | |
|---|---|---|---|---|---|---|---|---|---|
| Test substance was administered intrathecally to groups of 5 animals 30 minutes before the level of allodynia was again determined (post-treatment). 50 percent or greater (≥50%) inhibition relative to blank indicated possible analgesic activity. Note: (1) Δ Blank: Tactile Pressure [(PreLigation) - (Pre-Treatment)] (2) Δ Treatment: Tactile Pressure [(Post-Treatment) - (Pre-Treatment)] (3)% Inhibition = Δ Treatment/ Δ Blank x 100% (4) ITH:Intrathecal | | | | | | | | | |

**Table 6: Analgesia against Neuropathic Pain (Nerve Ligation in rats)**

| (60 Minutes) | | | | | | | | | |
|---|---|---|---|---|---|---|---|---|---|
| Treatment | Route | Dose | N | Tactile Pressure (g) | | | | | Inhibition (%) |
| | | | | Pre-Ligation | Pre-Treatment | Δ Blank | Post-Treatment | Δ Treatment | |
| Vehicle | ITH | 10 µg/animal | 1 | 32.1 | 11.7 | 20.4 | 6.2 | -5.5 | |
| (Sterile | | | 2 | 34.2 | 7.6 | 26.6 | 10.6 | 3.0 | |
| Saline) | | | 3 | 37.0 | 13.3 | 23.7 | 10.2 | -3.1 | |
| | | | 4 | 40.0 | 14.2 | 25.8 | 7.8 | -6.4 | |
| | | | 5 | 46.2 | 9.2 | 37.0 | 11.1 | 1.9 | |
| | | | Ave | 37.9 | 11.2 | 26.7 | 9.2 | -2.0 | -8 |
| Lofexidine HCl | ITH | 3.2µg/animal | 1 | 32.9 | 10.7 | 22.2 | 8.7 | -2.0 | |
| | | | 2 | 34.4 | 11.2 | 23.2 | 7.5 | -3.7 | |
| | | | 3 | 40.1 | 14.8 | 25.3 | 12.7 | -2.1 | |
| | | | 4 | 48.7 | 7.1 | 41.6 | 11.3 | 4.2 | |
| | | | 5 | 50.6 | 12.0 | 38.6 | 6.5 | -5.5 | |
| | | | Ave. | 41.3 | 11.2 | 30.2 | 9.3 | -1.8 | -6 |
| | ITH | 10µg/ animal | 1 | 37.8 | 12.1 | 25.7 | 20.5 | 8.4 | |
| | | | 2 | 40.9 | 10.9 | 30.0 | 15.1 | 4.2 | |
| | | | 3 | 35.6 | 6.2 | 29.4 | 36.5 | 30.3 | |
| | | | 4 | 37.0 | 11.0 | 26.0 | 23.2 | 12.2 | |
| | | | 5 | 39.6 | 11.3 | 28.3 | 8.9 | -2.4 | |
| | | | Ave. | 38.2 | 10.3 | 27.9 | 20.8 | 10.5 | 38 |

| | | | | | | | | | |
|---|---|---|---|---|---|---|---|---|---|
| Test substance was administered intrathecaily to groups of 5 animals 60 minutes before the level of allodynia was again determined (post-treatment). 50 percent or greater (http://www.dyana.info/Language/Phrases.htm50%) inhibition relative to blank indicated possible analgesic activity. Note: (1) Δ Blank: Tactile Pressure [(Pre-Ligation) - (Pre-Treatment)] (2) Δ Treatment Tactile Pressure [(Post Treatment) - (Pre-Treatment)] (3)% Inhibition = A Treatment/Δ Blank x 100% (4) ITH:Intrathecal | | | | | | | | | |

**Table 7: Analgesia against Neuropathic Pain (Nerve Ligation in rats)**

| (120 Minutes) | | | | | | | | | |
|---|---|---|---|---|---|---|---|---|---|
| Treatment | Route | Dose | N | Tactile Pressure (g) | | | | | Inhibition (%) |
| | | | | Pre-Ligation | Pre-Treatment | Δ Blank | Post-Treatment | Δ Treatment | |
| Vehicle | ITH | 10 µg/animal | 1 | 32.1 | 11.7 | 20.4 | 10.9 | -0.8 | |
| (Sterile | | | 2 | 34.2 | 7.6 | 26.6 | 8.4 | 0.8 | |
| Saline) | | | 3 | 37.0 | 13.3 | 23.7 | 10.3 | -3.0 | |
| | | | 4 | 40.0 | 14.2 | 25.8 | 11.1 | -3.1 | |
| | | | 5 | 46.2 | 9.2 | 37.0 | 9.0 | -0.2 | |
| | | | Ave. | 37.9 | 11.2 | 26.7 | 9.9 | -1.3 | -5 |
| Lofexidine HCl | ITH | 3.2µg/animal | 1 | 32.9 | 10.7 | 22.2 | 8.6 | -2.1 | |
| | | | 2 | 34.4 | 11.2 | 23.2 | 6.1 | -5.1 | |
| | | | 3 | 40.1 | 14.8 | 25.3 | 12.4 | -2.4 | |
| | | | 4 | 48.7 | 7.1 | 41.6 | 7.9 | 0.8 | |
| | | | 5 | 50.6 | 12.0 | 38.6 | 10.8 | -1.2 | |
| | | | Ave. | 41.3 | 11.2 | 30.2 | 9.2 | -2.0 | -7 |
| | ITH | 10µg/animal | 1 | 37.8 | 12.1 | 25.7 | 13.5 | 1.4 | |
| | | | 2 | 40.9 | 10.9 | 30.0 | 10.8 | -0.1 | |
| | | | 3 | 35.6 | 6.2 | 29.4 | 11.8 | 5.2 | |
| | | | 4 | 37.0 | 11.0 | 26.0 | 12.3 | 1.3 | |
| | | | 5 | 39.6 | 11.3 | 28.3 | 6.4 | -4.9 | |
| | | | Ave. | 38.2 | 10.3 | 27.9 | 11.0 | 0.6 | 2 |

| | | | | | | | | | |
|---|---|---|---|---|---|---|---|---|---|
| Test substance was administered intrathecaily to groups of 5 animals 60 minutes before the level of allodynia was again determined (post-treatment). 50 percent or greater (≥50%) inhibition relative to blank indicated possible analgesic activity. Note: (1) Δ Blank: Tactile Pressure [(Pre-Ligation) - (Pre-Treatment)] (2) Δ Treatment: Tactile Pressure [(Post-Treatment) - (Pre-Treatment)] (3) % Inhibition = Δ Treatment/Δ Blank x 100% (4) ITH:Intrathecal | | | | | | | | | |

### Conclusion

Lofexidine at 10 µg/animal by intrathecal administration demonstrated significant analgesic activity at 30 minutes post-injection; a moderate activity was also observed at 15 and 60 minutes time points (47% and 38% inhibition, respectively). At 3.2 µg/animal, lofexidine induced low levels of analgesic activity. The results are summarized below.

| Treatment | Route | Dose | %Inhibition | | | |
|---|---|---|---|---|---|---|
| | | | 15 min | 30 min | 60 min | 120 min |
| Vehicle | ITH | 10 µl/rat | -7 | -1 | -8 | -5 |
| Lofexidine HCl | ITH | 3.2 µg/rat | 4 | -4 | -6 | -7 |
| Lofexidine HCl | ITH | 10 µg/rat | 47 | 58 | 38 | 2 |

It is concluded that intrathecal injection of lofexidine HCl at 10 µg/animal is associated with analgesic activity in a rat model of neuropathic pain. The analgesic activity reached a peak at 30 minutes post-dosing and was no longer evident at 120 minutes post-dosing.

## Claims

1. Lofexidine or a pharmaceutically acceptable salt thereof for use in intraspinal administration to provide analgesia wherein said analgesia is for post-surgical pain and/or neuropathic pain.

2. Lofexidine or a pharmaceutically acceptable salt thereof for use according to any one of claim 1, in liposome-encapsulated form.

3. Lofexidine or a pharmaceutically acceptable salt thereof for use according to any one of claims 1 to 2, further comprising an opioid analgesic.

4. Lofexidine or a pharmaceutically acceptable salt thereof for use according to any one of claims 1 to 3, wherein said lofexidine is enantiomerically pure.

5. Lofexidine or a pharmaceutically acceptable salt thereof for use according to claim 4 wherein said lofexidine is (-)-lofexidine.

6. Lofexidine or a pharmaceutically acceptable salt thereof for use according to any one of claims 1 to 4, wherein said lofexidine is lofexidine hydrochloride.

7. Lofexidine or a pharmaceutically acceptable salt thereof for use according to claim 1, contained in an intraspinal syringe together with one or more physiologically acceptable carriers and/or diluents.

8. Use of lofexidine or a pharmaceutically acceptable salt thereof in the manufacture of a medicament for intraspinal administration to provide analgesia wherein said analgesia is for post-surgical pain and/or neuropathic pain.

## Patentansprüche

1. Lofexidin oder ein pharmazeutisch verträgliches Salz davon zur Verwendung für die intraspinale Verabreichung zum Bewirken einer Analgesie bei postoperativem Schmerz und/oder neuropathischem Schmerz.

2. Lofexidin oder ein pharmazeutisch verträgliches Salz davon zur Verwendung nach Anspruch 1 in einer mit Liposomen verkapselten Form.

3. Lofexidin oder ein pharmazeutisch verträgliches Salz davon zur Verwendung nach einem der Ansprüche 1 bis 2, ferner umfassend ein Opiod-Analgetikum.

4. Lofexidin oder ein pharmazeutisch verträgliches Salz davon zur Verwendung nach einem der Ansprüche 1 bis 3, wobei das Lofexidin enantiomerenrein ist.

5. Lofexidin oder ein pharmazeutisch verträgliches Salz davon zur Verwendung nach Anspruch 4, wobei das Lofexidin (-)-Lofexidin ist.

6. Lofexidin oder ein pharmazeutisch verträgliches Salz davon zur Verwendung nach einem der Ansprüche 1 bis 4, wobei das Lofexidin Lofexidin-Hydrochlorid ist.

7. Lofexidin oder ein pharmazeutisch verträgliches Salz davon zur Verwendung nach Anspruch 1, das zusammen mit einem oder mehreren physiologisch verträglichen Trägern und/oder Verdünnungsmitteln in einer intraspinalen Spritze enthalten ist.

8. Verwendung von Lofexidin oder einem pharmazeutisch verträglichen Salz davon für die Herstellung eines Medikaments zur intraspinalen Verabreichung zum Bewirken einer Analgesie bei postoperativem Schmerz und/oder neuropathischem Schmerz.

## Revendications

1. Lofexidine ou un de ses sels pharmaceutiquement acceptables, pour une utilisation dans une administration intrarachidienne afin d'obtenir une analgésie, où ladite analgésie est pour une douleur postchirurgicale et/ou une douleur neuropathique.

2. Lofexidine ou un de ses sels pharmaceutiquement acceptables pour une utilisation selon la revendication 1, sous une forme encapsulée dans un liposome.

3. Lofexidine ou un de ses sels pharmaceutiquement acceptables pour une utilisation selon l'une quelconque des revendications 1 à 2, comprenant en outre un analgésique opioïde.

4. Lofexidine ou un de ses sels pharmaceutiquement acceptables pour une utilisation selon l'une quelconque des revendications 1 à 3, où ladite lofexidine est énantiomériquement pure.

5. Lofexidine ou un de ses sels pharmaceutiquement acceptables pour une utilisation selon la revendication 4, où ladite lofexidine est (-)-lofexidine.

6. Lofexidine ou un de ses sels pharmaceutiquement acceptables pour une utilisation selon l'une quelconque des revendications 1 à 4, où ladite lofexidine est de l'hydrochlorure de lofexidine.

7. Lofexidine ou un de ses sels pharmaceutiquement acceptables pour une utilisation selon la revendication 1, contenu(e) dans une seringue pour injection intrarachidienne avec un ou plusieurs véhicules et/ou diluants physiologiquement acceptables.

8. Utilisation de lofexidine ou d'un de ses sels pharmaceutiquement acceptables dans la fabrication d'un médicament pour une administration intrarachidienne afin d'obtenir une analgésie, où ladite analgésie est pour une douleur postchirurgicale et/ou une douleur neuropathique.
